# EUROPÄISCHE PATENTSCHRIFT

(11) **EP 1 075 826 B1**
(45) Veröffentlichungstag und Bekanntmachung des Hinweises auf die Patenterteilung: **18.06.2003**
(21) Anmeldenummer: 00117059.6
(22) Anmeldetag: 09.08.2000
(51) Int. Cl.: A61F 2/16

(54) **Vorrichtung zur Faltung und Implantation einer Intraokularlinse oder Intracorneallinse**
Device for folding and implanting an intraocular lense or intracorneal lense
Dispositif pour plier et implanter une lentille intraoculaire ou intracornéen

(30) Priorität: 10.08.1999 DE 19937780
(43) Veröffentlichungstag der Anmeldung: 14.02.2001
(73) Patentinhaber: GenioVis GmbH, 52222 Stolberg (DE)
(72) Erfinder: Keune, Dirk, Dipl.-Ing. Dipl.- Wirt.-Ing., 52223 Stolberg (DE); Jansen, Matthias, Dipl.-Ing., 52223 Stolberg (DE)
(74) Vertreter: Bauer, Dirk, Dipl.-Ing. Dipl.-Kfm.

(56) Entgegenhaltungen:
- WO-A-98/37830
- WO-A-99/21514
- WO-A-99/33411
- US-A- 5 620 450

## Beschreibung

Die Erfindung betrifft eine Vorrichtung zur Faltung einer im ungefalteten Zustand vorliegenden Intraokularlinse oder Intracorneallinse sowie eine Vorrichtung zur Implantation einer gefalteten Intraokularlinse oder Intracorneallinse.

Aus der US 4,765,329 ist eine kombinierte Faltungs- und Implantationsvorrichtung bekannt, die eine Aufnahmeeinrichtung für die ungefaltete Linse und einen einen Hohlraum begrenzenden Faltungskörper aufweist, der einen Öffnungsquerschnitt besitzt, durch den die Linse von der Aufnahmeeinrichtung mittels einer Vorschubeinrichtung in den Faltungskörper schiebbar ist, wobei die größte lichte Weite des Öffnungsquerschnitts ungefähr 30 % bis 70 % des größten Durchmessers der Linse beträgt.

Die Vorschubeinrichtung ist in Form eines federbelasteten Dorns ausgeführt, der zunächst in axiale Richtung durch die als zylindrischer Körper ausgebildete Aufnahmeeinrichtung für die mittig vorgefaltete Linse hindurchgeführt wird und dabei die Linse in einen sich trichterförmig verjüngenden Übergangsbereich vorschiebt. Dort findet eine enge Faltung der Linse statt, so daß diese innerhalb eines als zylinderförmige Spitze ausgebildeten Faltungskörpers aufgenommen wird. Der Faltungskörper weist im Vergleich zur Aufnahmeeinrichtung einen wesentlich reduzierten Durchmesser auf und wird vollständig in eine Inzision in der Hornhaut des Auges eingeführt, wobei eine vordere Stirnseite der im Durchmesser vergrößerten Aufnahmeeinrichtung an der Hornhaut zur Anlage kommt und somit eine korrekte Position der Faltungs- und Implantationsvorrichtung sicherstellt. Nach Einsetzen der Faltungsvorrichtung mit ihrem Spitzenbereich in die Inzision erfolgt die eigentliche Implantation dadurch, daß der Dorn in die innere zylindrische Bohrung des Faltungskörpers vorgeschoben wird und dadurch die gefaltete Linse aus dem Faltungskörper herausbefördert. Innerhalb des Auges kann anschließend wieder eine Entfaltung der Linse stattfinden.

Ein Nachteil dieser bekannten Vorrichtung ist zum einen darin zu sehen, daß die Linse zum einen bereits in ihre Längsrichtung einmal vorgefaltet sein muß und daher in einer genau bestimmten Ausrichtung in die Aufnahmeeinrichtung eingelegt werden muß. Außerdem bedarf die Vorfaltung eines zusätzlichen Arbeitsschritts. Ferner ist durch das kleine Verhältnis von der Fläche der Vorschubeinrichtung und der Querschnittsfläche der Aufnahmeeinrichtung nicht immer sichergestellt, daß die Linse sicher in den Öffnungsquerschnitt des Faltungskörpers vorgeschoben werden kann.

Zum anderen ist es nachteilig, daß die Inzision in der Regel wesentlich größer als der Durchmesser des Spitzenteils des Faltungskörpers ausgeführt werden muß, da der Faltungskörper aufgrund seiner stumpfen zylindrischen Form ansonsten nicht ohne die Gefahr von Verletzungen in die Hornhaut eingeführt werden kann.

Die WO 99/21514 A offenbart eine Vorrichtung zur Faltung und Implantation einer Intraokularlinse gemäß dem Oberbegriff des Anspruchs 1. Vorgeschlagen wird, die in der Vorrichtung in distaler Richtung zu lagernde Haptik der Intraokularlinse in dem rohrförmigen Innenteil aufzunehmen. Das Innenteil wirkt beim Einbringen der Intraokularlinse in die Vorrichtung als Führungselement und vermeidet eine unerwünschte Verlagerung oder Faltung der Haptik in der Vorrichtung. Das Innenteil wird vor der Implantation der Linse in das Auge durch den distalen Endabschnitt aus der Vorrichtung entfernt.

Der Erfindung liegt die Aufgabe zugrunde, eine Faltungsvorrichtung vorzuschlagen, bei der die Linse ohne eine vorherige Faltung sicher in den gefalteten Zustand überführt werden kann.

Ausgehend von einer Vorrichtung zur Faltung einer Linse der eingangs beschriebenen Art, wird diese Aufgabe erfindungsgemäß dadurch gelöst, daß die Vorschubeinrichtung einen in Vorschubrichtung vorgelagerten Ansatz aufweist, auf dem die ungefaltete Linse ablegbar ist und der bei einer Vorschubbewegung der Vorschubeinrichtung zusammen mit der Linse in die Aufnahmeeinrichtung bewegbar ist.

Durch die Ablage der Linse in ihrem ungefalteten Zustand auf dem vorgelagerten Ansatz wird verhindert, daß sich die Linse während des Faltungsvorgangs in Bereiche unterhalb des Ansatzes ausdehnen kann. Vielmehr wird durch den Ansatz eine Ausrichtung der ungefaltet vorliegenden Linse vorgegeben, die garantiert, daß infolge der Vorschubbewegung eine kontrollierte Faltung eintritt. Die Faltung erfolgt dabei im Übergangsbereich des größeren Querschnitts der Aufnahmeeinrichtung in den dagegen reduzierten Öffnungsquerschnitt des Faltungskörpers und zwar in der Form, daß die Linse von beiden Seiten her zur Mitte umgebogen und zusammengerollt wird. Dabei fährt der Ansatz zusammen mit der sich faltenden Linse in den Öffnungsquerschnitt ein und unterstützt die Linse in einem Teilumfang. Vorzugsweise ist der Ansatz rinnenförmig, erstreckt sich in axiale Richtung der Vorschubeinrichtung und weist einen Außendurchmesser auf, der dem Innendurchmesser des Faltungskörpers entspricht.

Die Erfindung weiter ausgestaltend wird vorgeschlagen, daß der Faltungskörper und die Aufnahmeeinrichtung jeweils von einem Rohrabschnitt gebildet sind.

Ein kontrollierter und möglichst ungehinderter Übergang der Linse von der Aufnahmeeinrichtung in den Öffnungsquerschnitt wird erreicht, wenn ein Übergangsbereich zwischen der Aufnahmeeinrichtung und dem Faltungskörper konisch ausgebildet ist.

Die der Erfindung zugrundeliegende Aufgabe wird andererseits auch durch eine Vorrichtung zur Implantation einer gefalteten Intraokularlinse oder Intracorneallinse in das menschliche oder tierische Auge gelöst, die ein Außenteil aufweist, dessen distaler Endabschnitt in eine Inzision in der Hornhaut des Auges einbringbar ist und ein Innenteil besitzt, das koaxial zu dem Außenteil angeordnet und in dieses einschiebbar und dadurch gekennzeichnet ist, daß das Innenteil eine Vorrichtung zur Faltung einer im ungefalteten Zustand vorliegenden Intraokularlinse oder Intracorneallinse der oben beschriebenen Art umfasst.

Das Innenteil erfüllt hierbei eine Doppelfunktion und zwar die Funktion der Faltungsvorrichtung insgesamt sowie die Funktion des Vorschubelements beim Implantationsvorgang. Die zu implantierende Linse kann nach deren Faltung folglich im inneren Teil des Faltungskörpers verbleiben und wird bei dessen Vorschubbewegung ebenfalls auf dem Implantationsort vorgeschoben. Dabei erfolgt das Austreten der Linse durch Betätigung der Vorschubeinrichtung der Faltungseinrichtung..

Hierbei besitzt vorzugsweise das Außenteil die Form eines Spritzenkörpers und das Innenteil die Form eines Spritzenkolbens, wobei die Vorschubeinrichtung nur so weit innerhalb des Innenteils vorschiebbar ist, daß deren distale Stirnfläche mit der Stirnfläche des Innenteils fluchtet. Eine unbeabsichtigte Verletzung des Auges durch vortretende Teile der Vorschubeinrichtung ist hierdurch ebenso ausgeschlossen wie ein zu weites Vorschieben der Linse.

Die Erfindung wird nachfolgend anhand eines Ausführungsbeispiels einer Faltungsvorrichtung sowie zweier Implantationsvorrichtungen, die in der Zeichnung schematisch dargestellt sind, näher erläutert. Es zeigt:
- Figur 1: eine Faltungsvorrichtung mit einer Linse im ungefalteten Zustand;
- Figur 2: wie Figur 1, jedoch im gefalteten Zustand der Linse;
- Figur 3: eine Implantationsvorrichtung mit teilweise vorgeschobener Faltungsvorrichtung;
- Figur 4: wie Figur 3, jedoch mit vollständig vorgeschobener Faltungsvorrichtung
und
- Figur 5: eine Implantationsvorrichtung mit in ein Schlauchstück vorgeschobener Linse und teilweise vorgeschobener Faltungsvorrichtung.

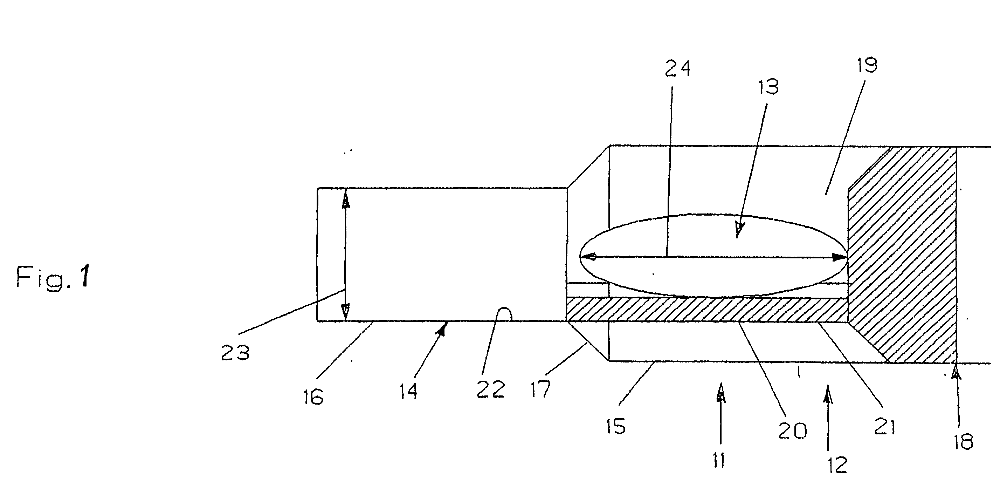

In Figur 1 ist schematisch dargestellt, wie eine Faltungsvorrichtung 11 aus einer Aufnahmeeinrichtung 12 für die ungefaltete Linse 13, in diesem Fall eine Intracorneallinse, und einem Faltungskörper 14 aufgebaut ist. Die Aufnahmeeinrichtung 12 wird von einem zylindrischen Rohrabschnitt 15 und der Faltungskörper 14 von einem ebenfalls zylindrischen, jedoch einen kleineren Durchmesser aufweisenden Rohrabschnitt 16 gebildet. Ein Übergangsbereich 17 zwischen den beiden Rohrabschnitten 15 und 16 ist konisch ausgebildet.

Die Faltungsvorrichtung 11 ist des weiteren mit einer Vorschubeinrichtung 18 versehen, die mit ihrem Außendurchmesser an den Innendurchmesser des Rohrabschnitts 15 angepaßt und an ihrer vorderen Stirnseite 19 mit einem rinnenförmigen Ansatz 20 versehen ist.

Die Vorschubeinrichtung 18 ist koaxial in dem Rohrabschnitt 15 verschiebbar gelagert, wobei der Ansatz 20 so angeordnet ist, daß seine äußere Mantelfläche 21 an der inneren Mantelfläche 22 des Rohrabschnitts 16 im wesentlichen zur Anlage kommt

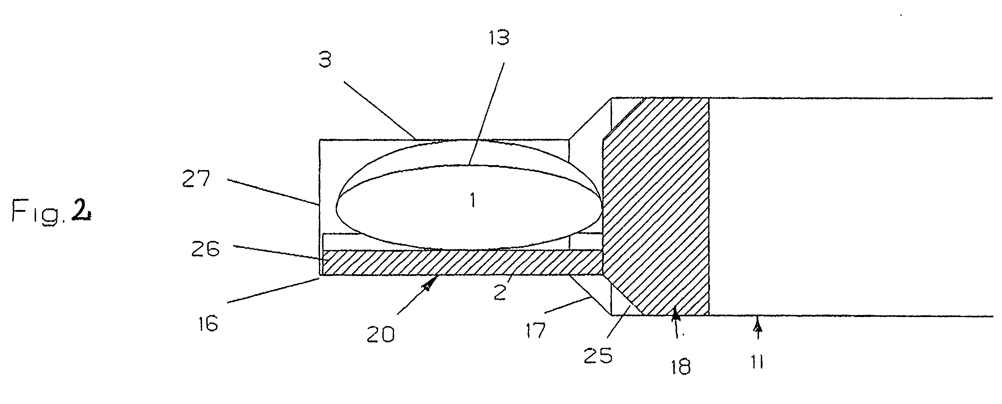

In Figur 2 ist die Vorschubeinrichtung 18 in einer vorgeschobenen Position und die Linse 13 im gefalteten Zustand dargestellt. Eine Faltung der Linse 13 tritt beim Vorschub der Vorschubeinrichtung 18 deshalb automatisch ein, weil der Innendurchmesser 23 des Rohrabschnitts 16 nur ca. 30 % des größten Durchmessers 24 der Linse 13 beträgt. Der konische Übergangsbereich 17 bewirkt beim Kontakt mit den daran anstoßenden Oberflächenbereichen der Linse 13 eine sanfte Ablenkung nach innen, so daß Beschädigungen des Linsenmaterials auszuschließen sind.

In Figur 2 hat die Vorschubeinrichtung 18 noch nicht vollständig ihre Endposition erreicht. Diese liegt dann vor, wenn die konische Fläche 25 der Vorschubeinrichtung 18 an der Fläche des Übergangsbereichs 17 zur Anlage kommt, wobei in dieser Position die Stirnseite 26 des Ansatzes 20 mit der Stirnseite 27 des Rohrabschnitts 16 fluchtet.

Figur 3 zeigt eine Implantationsvorrichtung 28, die aus einem Außenteil 29 in Form eines Spritzenkörpers sowie einem darin koaxial verschiebbar angeordneten Innenteil besteht, bei dem es sich um die Faltungsvorrichtung 11 handelt. In der Faltungsvorrichtung 11 befindet sich innerhalb des vorderen Rohrabschnitts 16 die gefaltete Linse 13. Die Vorschubeinrichtung 18 mit dem rinnenförmigen Ansatz 20 gemäß den Figuren 1 und 2 ist in Figur 3 durch eine Vorschubeinrichtung 18' ersetzt, die einen zylindrischen Ansatz 20' mit geschlossener, kreisförmiger Stirnfläche 26' aufweist. Der Durchmesser 30 des Ansatzes 20' stimmt im wesentlichen mit dem Innendurchmesser 23 des Rohrabschnitts 16 überein und eignet sich daher zum Vorschieben der Linse 13.

Das Außenteil 29 weist als distalen Endabschnitt 31 ein radial dehnbares Schlauchstück 32 auf, das im Ruhezustand einen Innendurchmesser 33 aufweist, der kleiner als der Außendurchmesser 23' des Rohrabschnitts 16 ist.

In Figur 3 ist das Innenteil in Form der Faltungsvorrichtung 11 bereits um einen gewissen Weg 34 in das Schlauchstück 32 unter Aufdehnung dessen Durchmessers eingeschoben.

Ausgehend von einem derartigen Zustand, wird der vordere ungedehnte Abschnitt des Schlauchstücks 32 in eine zuvor hergestellte Inzision in der Hornhaut des Auges eingesetzt. Anschließend wird die Faltungseinrichtung 11 weiter in das Außenteil 29 eingeschoben, wodurch auch der vordere Abschnitt des Schlauchstücks 32 sowie das diesen unmittelbar umgebende Hornhautgewebe im Bereich der Inzision elastisch gedehnt wird.

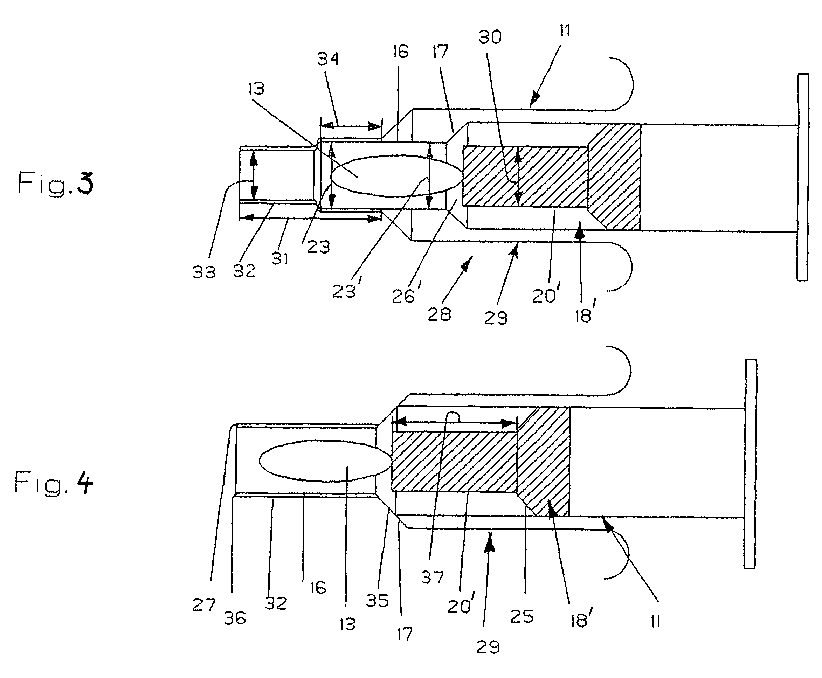

In Figur 4 ist der Zustand dargestellt, in dem die Faltungsvorrichtung 11 so weit in das Außenteil 29 eingeschoben ist, daß die Faltungsvorrichtung 11 mit ihrem konischen Übergangsbereich 17 an der Fläche eines ebenfalls konischen Übergangsbereichs 35 des Außenteils 29 zur Anlage kommt. In dieser Position ist das Schlauchstück 32 über seine gesamte Länge aufgedehnt. Des weiteren fluchtet die Stirnfläche 36 des Außenteils 29 mit der Stirnfläche 27 der Faltungsvorrichtung 11. Ein ungewollt weites Vorschieben der Faltungsvorrichtung 11 wird auf diese Weise verhindert.

In dem in Figur 4 dargestellten Zustand ist die Linse 13 bereit zur Implantation. Die Inzision ist auf das erforderliche Maß aufgedehnt, und durch Verlagerung der Vorschubeinrichtung 18' in der Faltungsvorrichtung 11 kann die Linse 13 nunmehr aus dem Rohrabschnitt 16 herausgeschoben und am Implantationsort plaziert werden. Die Länge 37 des Ansatzes 20' ist dabei so bemessen, daß bei Anlage der konischen Fläche 25 der Vorschubeinrichtung 18' an der Fläche des Übergangsbereichs 17 die Stirnfläche 26' mit den Stirnflächen 27 des Rohrabschnitts 16 und 36 des Außenteils 29 fluchtet. Eine Verletzungsgefahr durch eine unbeabsichtigt weit vorgeschobene Vorschubeinrichtung 18'besteht somit nicht.

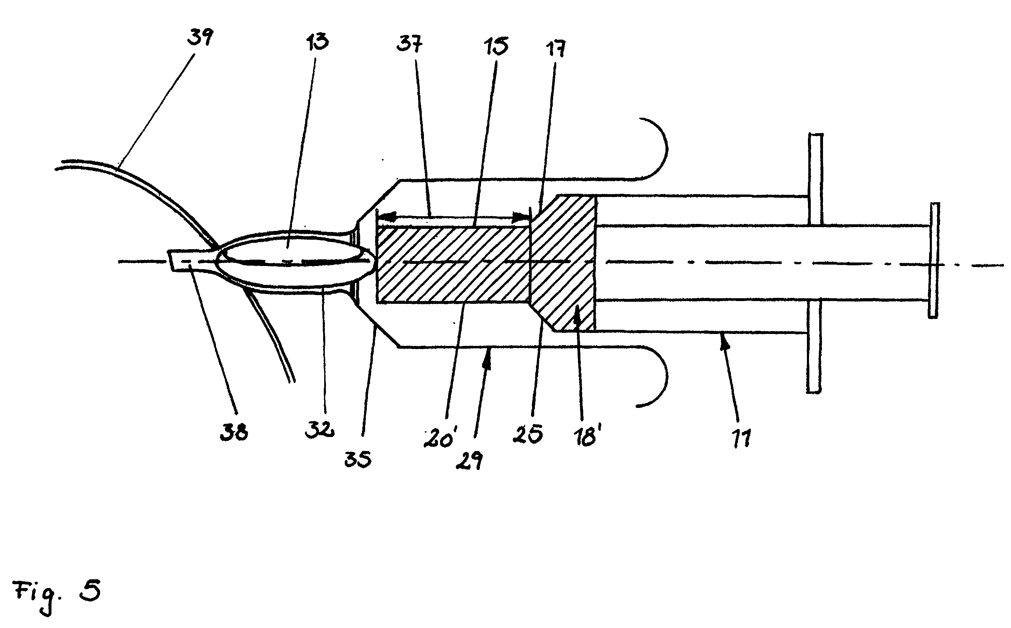

In Figur 5 ist dargestellt, wie unter Verwendung derselben Faltungsvorrichtung 11 und derselben Implantationsvorrichtung 28 eine andere Vorgehensweise beim Implantieren gewählt werden kann: Zunächst wird die Linse unter Verwendung der Faltungsvorrichtung 11 gemäß den in den Figuren 1 und 2 beschriebenen Schritten in den gefalteten Zustand überführt. Das Innenteil der Implantationsvorrichtung 28 in Form der Faltungsvorrichtung 11 wird nunmehr ungefähr so weit in das Außenteil 29 eingeschoben, daß sich die distale Stirnseite der Faltungsvorrichtung 11 nahezu im Bereich des Schlauchansatzes befindet. Anschließend wird durch Vorschieben der Vorschubeinrichtung 18' die Linse 13 aus dem Faltungskörper, das heißt dem Rohrabschnitt 15 herausgeschoben und tritt dabei sukzessive in das Schlauchstück 32 ein, das sich im ungedehnten Ausgangszustand entgegen der Implantationsrichtung trompetenförmig in seinem Anschlußbereich an das Außenteil 29 erweitert.

Wie sich der Figur 5 entnehmen läßt, paßt sich das Schlauchstück 32, dessen Endbereich 38 zuvor bereits in eine angepaßte kleine Inzision in der Hornhaut 39 des Auges eingesetzt wurde, der Form der gefalteten Linse an und wird somit aufgeweitet. Die eigentliche Implantation erfolgt nunmehr dadurch, daß in einem nächsten Schritt die gesamte Faltungsvorrichtung 11 als Innenteil in das Außenteil 29 der Implantationsvorrichtung 28 vorgeschoben wird, wodurch das Schlauchstück 32 im Bereich hinter der Linse 13 auf den Durchmesser des distalen Endabschnitts der Faltungsvorrichtung 11 erweitert wird. Diese größere Erweiterung des Schlauchstücks 32 erstreckt sich jedoch nicht bis in den Bereich der Inzision in der Hornhaut 39. Vielmehr wird die Faltungsvorrichtung 11 nur so weit vorgeschoben, daß die gefaltete Linse 13 mit ihrem größten Durchmesser durch die eingeschnittene Hornhaut hindurchgetreten ist und sich infolge ihres sich verjüngenden Endabschnitts nahezu selbständig vollständig in das Auge bewegt.

Während bei der Implantationsweise, wie sie in den Figuren 3 und 4 dargestellt ist, zunächst die Faltungsvorrichtung 11 (mit nicht vorgeschobener Vorschubeinrichtung 18') mit ihrem distalen Endabschnitt in das dehnbare Schlauchstück 32 vorgeschoben wird, und erst dann durch das bereits erweiterte Schlauchstück 32 die Linse 13 durch die dilatierte Hornhaut hindurchgeschoben wird, erfolgt bei der in Figur 5 veranschaulichten Vorgehensweise zunächst ein Vorschieben der Linse 13 aus dem Faltungskörper 14 heraus unmittelbar in das Schlauchstück 32, indem die Vorschubeinrichtung 18' vorgeschoben wird, und die eigentliche Implantation erst infolge des vollständigen Einschiebens des Innenteils (Faltungskörper 14 zusammen mit der Vorschubeinrichtung 18') in das Außenteil 29, wodurch eine Dehnung des Schlauchstücks 32 hinter der gefalteten Linse 13 durch das distale Endstück der Faltungsvorrichtung 11 bewirkt wird. Bei letzterer Vorgehensweise wird die Inzision in der Hornhaut 39 lediglich auf die Einhüllende des von der Linse 13 aufgeweiteten Schlauchstücks 32 aufgeweitet, wohingegen lediglich der hintere Abschnitt des Schlauchstücks 32, der sich außerhalb der Hornhaut 39 befindet, infolge einer Vorschubbewegung des Innenteils auf den Durchmesser von dessen distalen Endstücks aufgeweitet wird.

## Patentansprüche

1. Vorrichtung zur Faltung einer im ungefalteten Zustand vorliegenden Intraokularlinse oder Intracorneallinse mit einer Aufnahmeeinrichtung (12) für die ungefaltete Linse (13) und einem einen Hohlraum begrenzenden Faltungskörper (14), der einen Öffnungsquerschnitt aufweist, durch den die Linse (13) von der Aufnahmeeinrichtung (12) mittels einer Vorschubeinrichtung (18) in den Faltungskörper (14) schiebbar ist, wobei die größte lichte Weite des Öffnungsquerschnitts ungefähr 30 % bis 70 % des größten Durchmessers der Linse (13) beträgt, **dadurch gekennzeichnet, daß** die Vorschubeinrichtung (18) einen in Vorschubrichtung vorgelagerten Ansatz (20) aufweist, auf dem die ungefaltete Linse (13) ablegbar ist und der bei einer Vorschubbewegung der Vorschubeinrichtung (18) zusammen mit der Linse (13) durch den Öffnungsquerschnitt bewegbar ist.

2. Vorrichtung nach Anspruch 1, **dadurch gekennzeichnet, daß** der Ansatz (20) rinnenförmig ist, sich in axiale Richtung der Vorschubeinrichtung (18) erstreckt und einen Außendurchmesser aufweist, der dem Innendurchmesser (23) der Aufnahmeeinrichtung entspricht.

3. Vorrichtung nach Anspruch 1 oder 2, **dadurch gekennzeichnet, daß** der Faltungskörper (14) und die Aufnahmeeinrichtung (12) jeweils von einem Rohrabschnitt (15, 16) gebildet sind.

4. Vorrichtung nach einem der Ansprüche 1 bis 3, **dadurch gekennzeichnet, daß** ein Übergangsbereich (17) zwischen der Aufnahmeeinrichtung (12) und dem Faltungskörper (14) konisch ausgebildet ist.

5. Vorrichtung zur Implantation einer gefalteten Intraokularlinse oder Intracorneallinse in das menschliche oder tierische Auge mit einem Außenteil (29), dessen distaler Endabschnitt (31) in eine Inzision in der Hornhaut des Auges einbringbar ist, und einem koaxial zu dem Außenteil (29) angeordneten und in dieses einschiebbaren Innenteil, **dadurch gekennzeichnet, daß** das Innenteil eine Vorrichtung zur Faltung einer im ungefalteten Zustand vorliegenden Intraokularlinse oder Intracorneallinse nach einem der vorgenannten Ansprüche umfasst.

## Claims

1. Device for bending an intraocular or intracorneal lens that is present in the uncurled state, with a device (12) for taking up the uncurled lens (13) and a bending body (14), adjoining a cavity, which has an opening with a cross-sectional area through which the lens (13) can be pushed into the bending body (14), by means of a feeder device (12), by the uptake device (12), whereby the greatest unobstructed breadth of the opening cross-sectional area is approximately 30 - 70% of the greatest diameter of the lens (13), **characterized in that** the feeder device (18) has a rest (20), introduced into the feeder device (18), on which the uncurled lens (13) can be placed and which can be moved, with a feeding movement of the feeder device (18), together with the lens (13), through the transverse section of the opening.

2. Device in accordance with claim 1, **characterized in that** the rest (20) is in the form of a channel, **in that** it extends in the axial direction of the feeder device (18), and **in that** it has an outer diameter that corresponds to the inner diameter (23) of the uptake device.

3. Device in accordance with claim 1 or 2, **characterized in that** the bending body (14) and the uptake device (12) are formed from one pipe section (15, 16).

4. Device in accordance with one of claims 1 to 3, **characterized in that** a transition area (17) is conically developed between the uptake device (12) and the bending body (14).

5. Device for implanting a curled intraocular or intracorneal lens in the human or animal eye, with an outer section (29), the distal end section (31) of which can be inserted into an incision in the cornea, and an inner section that is disposed coaxially in relation to the outer section (29) and can be inserted into the outer section (29), **characterized in that** the inner section includes, in accordance with one of the preceding claims, a device for bending an intraocular or intracorneal lens in the uncurled state.

## Revendications

1. Dispositif de pliage d'une lentille intraoculaire ou d'une lentille intracornéenne se présentant à l'état déplié, comprenant un dispositif (12) de réception de la lentille (13) dépliée et une pièce (14) de pliage délimitant une cavité et ayant une section transversale d'ouverture par laquelle la lentille (13) peut être poussée au moyen d'un dispositif (18) d'avance du dispositif (12) de réception dans la pièce (14) de pliage, la plus grande dimension de la section transversale de l'ouverture représentant environ de 30 % à 70 % du plus grand diamètre de la lentille (13), **caractérisé en ce que** le dispositif (18) d'avance a un prolongement (20) placé en avant dans le sens d'avance, sur lequel la lentille (13) dépliée peut être posée et qui peut, lors d'un mouvement d'avance du dispositif (18) d'avance, être déplacé ensemble avec la lentille (13) dans la section transversale d'ouverture.

2. Dispositif suivant la revendication 1, **caractérisé en ce que** le prolongement (20) est en forme de goulotte, s'étend dans la direction axiale du dispositif (18) d'avance et a un diamètre extérieur qui correspond au diamètre (23) intérieur du dispositif de réception.

3. Dispositif suivant la revendication 1 ou 2, **caractérisé en ce que** la pièce (14) de pliage et le dispositif (12) de réception sont formés respectivement par un tronçon (15, 16) de tube.

4. Dispositif suivant l'une des revendications 1 à 3, **caractérisé en ce qu'**une partie (17) de transition entre le dispositif (12) de réception et la pièce (14) de pliage est connue.

5. Dispositif d'implantation d'une lentille intraoculaire ou d'une lentille intracornéenne pliée dans l'oeil de l'homme ou de l'animal, comprenant une partie (29) extérieure dont le tronçon (31) d'extrémité distale peut être introduit dans une incision de la cornée de l'oeil et une partie intérieure disposée coaxialement à la partie (29) extérieure et pouvant être introduite dans celle-ci, **caractérisé en ce que** la partie intérieure comprend un dispositif de pliage d'une lentille intraoculaire ou d'une lentille intracornéenne se présentant à l'état déplié suivant l'une des revendications précédentes.
